Europäisches Patentamt

European Patent Office

Office européen des brevets

(19)

(11) Publication number: **0 024 842**
**B1**

# EUROPEAN PATENT SPECIFICATION

(12)

(45) Date of publication of patent specification: **05.10.83**

(21) Application number: **80302723.4**

(22) Date of filing: **08.08.80**

(51) Int. Cl.³: **C 07 D 409/10,**
**A 61 K 31/415**
**//(C07D409/10, 235/32,**
**333/20)**

(54) Methyl (5-(2-thienylhydroxymethyl)-1H-benzimidazol-2-yl) carbamate, process for its preparation and compositions containing it.

(30) Priority: **23.08.79 US 69199**

(43) Date of publication of application:
**11.03.81 Bulletin 81/10**

(45) Publication of the grant of the patent:
**05.10.83 Bulletin 83/40**

(84) Designated Contracting States:
**AT BE CH DE FR GB IT LI LU NL SE**

(56) References cited:
**US - A - 4 032 536**

(73) Proprietor: **SMITHKLINE BECKMAN**
**CORPORATION**
**1 Franklin Plaza**
**Philadelphia Pennsylvania 19101 (US)**

(72) Inventor: **Chow, Alfred Wen-Jen**
**256 Upper Gulph Road**
**Radnor Pennsylvania 19087 (US)**
Inventor: **Gyurik, Robert John**
**Dowlin Forge Road, RD-2**
**Downington Pennsylvania 19335 (US)**
Inventor: **Parish, Roger Cook**
**184 Redwood Road**
**King of Prussia Pennsylvania 19406 (US)**

(74) Representative: **Claisse, John Anthony, Dr. et al,**
**P.O. Box 39 Welwyn Garden City**
**Hertfordshire AL7 1EY (GB)**

# 0 024 842

Methyl [5-(2-thienylhydroxymethyl)-1H-benzimidazol-2-yl]carbamate, process
for its preparation and compositions containing it

This invention concerns a new 5-substituted 1H-benzimidazol-2-yl-carbamate which has potent antimitotic activity. It also has anthelmintic activity.

Methyl 5-[(2-thenoyl)-1H-benzimidazol-2-yl]carbamate together with its anthelmintic activity is described in U.S. Patent Specification No. 3,657,267 (see Example XIV). Medical compositions which contain the same compound as active ingredient and which have activity against malignant neoplasms are described in German patent application 2,608,796. Other heterocyclic ketones and carbinols in the 2-carbamyl benzimidazole series are described in U.S. Patent Specification No. 4,026,936.

According to the invention there is provided methyl [5-(2-thienylhydroxymethyl)-1H-benzimidazol-2-yl]carbamate.

Methyl [5-(2-thienylhydroxymethyl)-1H-benzimidazol-2-yl]carbamate can be prepared by reduction of methyl [5-(2-thenoyl)-1H-benzimidazol-2-yl]carbamate under very specific reaction conditions. The reduction can be effected using sodium borohydride, usually in excess, in an aqueous alcoholic solvent such as aqueous methanol, ethanol or isopropanol. The reaction is particularly conveniently carried out with a 2/1 isopropanol-water mixture. Heating gives good yields, for example at 30—75°C, especially 55—60°C or at the boiling point of the reaction mixture, for from $\frac{1}{4}$ to 3 hours. The thenoyl starting material should be substantially soluble in the solvent system at the temperature of reaction.

The reduction at first proved unusually difficult because of the low solubility of the thenoyl starting material in many common solvents and the poisoning of hydrogenation catalysts by the presence of the sulfur atom in the structure of the starting material. The specific reduction conditions outlined above using sodium borohydride unexpectly gave good yields of the desired compound.

The desired product can be recovered by normal methods, such as cooling and then removing the solvent. Little further purification is usually necessary beyond washing the product.

The compound of this invention can exist as optical isomers. These can be separated by fractional crystallization techniques of the art; however, the mixture as formed in the above described process is preferred because of lower chemical cost.

The compound of this invention is a very active antimitotic agent. Various test procedures which evaluate antimitotic activity and the relevance of antimitotic activity to clinical use are described by P. Delatour and Y. Richard, Therapie, 31, 505—515 (1976). The compound of this invention was tested by P. Delatour according to the procedure described in detail in his publication in Therapie. The compound of this invention (compound I) was found to have a high degree of embryotoxic activity in the rat when administered orally to pregnant Spraque-Dawley rats daily from the 8th to the 15th day of gestation compared with its thenoyl congener, a known antimitotic agent (compound II).

| Compound | Dose mg/kg/day | # Gestating Females | # Implantations | # Fetus | Embryotoxicity % |
|---|---|---|---|---|---|
| I | 5.05 | 4 | 46 | 0 | 100 |
| | 3.03 | 4 | 58 | 51 | 12.06 |
| II | 30 | 3 | 28 | 0 | 100 |
| | 15 | 2 | 30 | 0 | 100 |
| | 7.5 | 3 | 40 | 0 | 100 |
| | 5.02 | 4 | 57 | 55 | 3.51 |
| | 5.02 | 4 | 39 | 33 | 15.4 |
| | 5.02 | 2 | 19 | 13 | 31.6 |

The data show that the carbinol compound of this invention has a 100% embryotoxic effect at a dose of 5.05 mg/kg/day whereas the prior art compound (II) which is the keto analogue of the carbinol (I) has only a low degree of activity at the same molar dose.

2

| Compound | Oral Dose Per Day | Foet./ Implant | % |
|---|---|---|---|
| I <br> (structure: thiophene-CH(OH)-benzimidazole-NHCO$_2$CH$_3$) | 5.05 mg/kg | 0/47 | 100 |
| II <br> (structure: thiophene-C(O)-benzimidazole-NHCO$_2$CH$_3$) | 5.02 mg/kg | 55/57 | 3.5 |

These data in another test demonstrate that the compound of this invention (I) has a 100% embryotoxic effect in rats while an equimolar quantity of the prior art keto compound (II) had only a 3.5% effect.

The compound of this invention can be formulated into pharmaceutical compositions for inducing antimitotic activity using a suitable carrier. The compositions will usually be in a form suitable for internal administration, preferably orally, to subjects in need of antimitotic treatment. Normally the compositions will be in the form of capsules, tablets, troches or liquid suspensions containing a pharmaceutically effective quantity of the compound of this invention, in general in an amount of from 10 to 150 mg per dosage unit. The compositions will usually be administered from 1—5 times daily. Liquid suspension or solutions which can be used for parenteral injection and in general when in dosage unit form they will contain similar quantities of the compound of this invention per dosage unit. Localized injection may be of particular utility.

Following is an Example of the chemical preparation of the compound of this invention.

Example 1

A mixture of 2.0 g of methyl N-[5-(thenoyl)-2-benzimidazolyl]carbamate, 80 ml of water and 120 ml of isopropanol was heated to 50°C at which point 2.0 g of solid sodium borohydride were added at once, with stirring. The mixture was heated to 60°C for 15 minutes. After a further reaction period of 30 minutes at 60°C, the reaction mixture was cooled. The isopropanol was removed under reduced pressure. The white product was removed by filtration, washed well with water and dried *in vacuo* to give 1.7 g of methyl [5-(2-thienylhydroxymethyl)-1H-benzimidazol-2-yl]carbamate, m.p. 300°C.

C$_{14}$H$_{14}$N$_3$O$_3$S
Calculated:  C, 55.43;  H, 4.32;  N, 13.85
Found:  C, 55.41;  H, 4.30;  N, 14.15

R$_f$ (90 : 10 : 1 chloroform; methanol; 40% ammonium hydroxide on silicon dioxide = 0.50 (R$_f$ = 0.66 for ketone).
N.M.R. (DMSO—d$_6$ S(1) 3.75; S(1) 5.95; m(6) 6.80—7.46.

Example 2

The following ingredients are mixed, filled into a gelatin capsule and administered orally from 1—5 times daily to a patient in need of antimitotic treatment.

| | |
|---|---|
| methyl N-[5-(2-thienylhydroxymethyl)-1H-benzimidazol-2-yl]carbamate | 75 mg. |
| lactose | 100 mg. |
| starch | 25 mg. |
| talcum | 50 mg. |

Claims

1. Methyl [5-(2-thienylhydroxymethyl)-1H-benzimidazol-2-yl]carbamate.
2. A process for preparing methyl [5-(2-thienylhydroxymethyl)-1H-benzimidazol-2-yl]carbamate

**O 024 842**

which comprises reducing methyl [5-(2-thenoyl-1H-benzimidazole-2-yl]carbamate with sodium borohydride.

3. Methyl [5-(2-thienylhydroxymethyl)-1H-benzimidazol-2-yl]carbamate for use as an antimitotic agent.

4. A pharmaceutical or veterinary composition comprising methyl [5-(2-thienylhydroxymethyl)-1H-benzimidazol-2-yl]carbamate and a carrier.

**Revendications**

1. [(Thiényl-2 hydroxyméthyl)-5 1H-benzimidazolyl-2]carbamate de méthyle.

2. Procédé de préparation du [(thiényl-2 hydroxyméthyl)-5 1H-benzimidazolyl-2]carbamate de méthyle qui consiste à réduire le [(thénoyl-2)-5 1H-benzimidazolyl-2]carbamate de méthyle par le borohydrure de sodium.

3. [(Thiényl-2 hydroxyméthyl)-5 1H-benzimidazolyl-2]carbamate de méthyle pour usage comme agent antimitotique.

4. Composition pharmaceutique ou vétérinaire qui contient le [(thiényl-2 hydroxyméthyl)-5 1H-benzimidazolyl-2]carbamate de méthyle et un véhicule ou excipient.

**Patantansprüche**

1. Methyl-[5-(2-thienylhydroxymethyl)-1H-benzimidazol-2-yl]-carbamat.

2. Verfahren zur Herstellung von Methyl-[5-(2-thienylhydroxymethyl)-1H-benzimidazol-2-yl]-carbamat, dadurch gekennzeichnet, daß man Methyl-[5-(2-thenoyl)-1H-benzimidazol-2-yl]-carbamat mit Natriumborhydrid reduziert.

3. Methyl-[5-(2-thienylhydroxymethyl)-1H-benzimidazol-2-yl]-carbamat zur Verwendung als antimitotisches Mittel.

4. Pharmazeutische Zusammensetzung für Mensch oder Tier, enthaltend Methyl-[5-(2-thienyl-hydroxymethyl)-1H-benzimidazol-2-yl]-carbamat sowie einen Trägerstoff.

4